# FASCICULE DE BREVET EUROPEEN

(11) **EP 3 280 458 B1**
(45) Date de publication et mention de la délivrance du brevet: **09.06.2021**
(21) Numéro de dépôt: 16718269.0
(22) Date de dépôt: 08.04.2016
(51) Int. Cl.: A61L 2/18, A61L 2/20, A23L 3/3409, A23B 9/18

(54) **PROCEDE ET DISPOSITIF DE TRAITEMENT A L'OZONE EN CONTINU DE PRODUITS DIVISES COMPRENANT DES MOYENS DE CONVOYAGE ET DE VIBRATION DES PRODUITS**
VERFAHREN UND VORRICHTUNG ZUR KONTINUIERLICHEN OZONBASIERTEN BEHANDLUNG VON TEILCHENFÖRMIGEN PRODUKTEN, MIT MITTELN ZUM FÖRDERN UND VIBRIEREN DIESER PRODUKTE
METHOD AND DEVICE FOR THE CONTINUOUS OZONE-BASED TREATMENT OF PARTICULATE PRODUCTS, COMPRISING MEANS FOR CONVEYING AND VIBRATING SAID PRODUCTS

(30) Priorité: 10.04.2015 FR 1553146
(43) Date de publication de la demande: 14.02.2018
(73) Titulaire: E.T.I.A. - EVALUATION TECHNOLOGIQUE, INGENIERIE ET APPLICATIONS, 60201 Compiègne cedex (FR)
(72) Inventeur: LEPEZ, Olivier, 60260 Lamorlaye (FR); SAJET, Philippe, 60610 Lacroix Saint-Ouen (FR); GUZUN, Tatiana, 60200 Compiègne (FR)
(74) Mandataire: Decorchemont, Audrey Véronique Christèle
(86) Numéro de dépôt international: PCT/EP2016/057812
(87) Numéro de publication internationale: WO 2016/162511

(56) Documents cités:
- WO-A2-2009/019570
- GB-A- 2 517 022
- JP-A- H11 235 377

## Description

La présente invention concerne un procédé de traitement de produits se présentant sous forme de solides divisés. Les produits divisés sont notamment, bien que non exclusivement, des produits alimentaires tels qu'épices, herbes, aromates, légumes déshydratés, fruits secs, produits laitiers en poudre ou encore les plantes aromatiques ou médicinales.

### ARRIERE-PLAN TECHNOLOGIQUE

De par ses propriétés stérilisantes, l'ozone est aujourd'hui couramment utilisé dans le domaine de la débactérisation de produits alimentaires.

Les dispositifs actuels de débactérisation à base d'ozone présentent usuellement une enceinte dans laquelle on vient agencer le produit à traiter avant d'y introduire de l'ozone. On laisse alors reposer le produit jusqu'à plusieurs heures dans cette atmosphère ozonisée avant de retirer le produit.

Toutefois, il s'avère que la simple immersion des grains dans une atmosphère ozonisée ne permet pas toujours d'obtenir une débactérisation suffisamment importante.

Pour améliorer la débactérisation, il a ainsi été mis au point des dispositifs dans lequel le produit est mis en contact avec l'ozone à différentes reprises.

On connait ainsi le dispositif appelé « Oxygreen » (marque déposée) décrit dans le livre « Ozone in Food Processing » écrit par Colm O'Donnell, Brijesh K. Tiwari, P. J. Cullen et Rip G. Rice et publié par John Wiley & Sons. Le dispositif « Oxygreen » permet le traitement de grains destinés à être utilisés pour la fabrication de farines. Les grains sont placés dans une enceinte comportant une zone de traitement à l'ozone en partie basse et une vis sans fin assurant une remontée du produit à traiter de ladite partie basse à la partie haute de l'enceinte : le produit s'écoule ainsi naturellement de la partie haute à la partie basse pour atteindre la zone de traitement à l'ozone avant d'être remonté par la vis sans fin jusqu'en partie haute où il s'écoule de nouveau en direction de la partie basse. Le produit est ainsi soumis de façon discontinue mais répétitive à l'action de l'ozone lors de son passage dans la partie basse de l'enceinte.

Bien qu'un tel procédé permette d'assurer une meilleure débactérisation du produit, il s'avère toutefois relativement délicat à mettre en œuvre avec notamment un risque d'oxydation du produit par l'ozone.

Le document GB 2 517 022 propose un traitement d'un produit consistant à émettre des radiations ultraviolet pulsées dans une enceinte ouverte sur l'extérieur.

Le document CN 1 294 858, le document JP S52 3871 et le document WO 2009/019570 illustrent d'autres dispositifs de l'art antérieur.

Le document JP H11 235377 propose une installation de traitement continu d'un produit sous forme de poudre ou de graine. L'installation comprend ainsi une vis pour l'avancée du produit dans l'enceinte et des moyens de chauffage du produit dans une atmosphère pressurisée.

### OBJET DE L'INVENTION

La présente invention a pour objet un procédé de traitement d'un produit sous forme de solides divisés qui obvie au moins en partie aux inconvénients précités. La présente invention a également pour objet un dispositif de traitement d'un produit sous forme de solides divisés correspondant.

### DEFINITION GENERALE DE L'INVENTION

Le problème précité est résolu conformément à l'invention grâce à un procédé de traitement en continu à l'ozone d'un produit sous forme de solides divisés comportant les étapes de :
- introduire le produit dans une enceinte fermée dans laquelle règne une atmosphère ozonisée sous pression, l'enceinte étant conformée en un serpentin s'enroulant autour d'un arbre central,
- convoyer le produit dans l'enceinte selon un mouvement continu entre une entrée de l'enceinte et une sortie de l'enceinte de sorte que le produit se trouve continuellement dans l'atmosphère ozonisée lors de son convoyage dans l'enceinte, le produit étant convoyé dans l'enceinte par des moyens de vibration faisant vibrer l'enceinte,
- expulser le produit hors de l'enceinte par la sortie de l'enceinte après un unique passage du produit à travers l'enceinte.

Ainsi, contrairement aux systèmes antérieurs rappelés plus haut, qui fonctionnent selon un traitement à l'ozone de façon discontinue éventuellement répétitive, le procédé selon l'invention met en œuvre un traitement à l'ozone de façon continue : en même temps que le produit est déplacé dans l'enceinte, le produit se trouve continuellement en contact avec l'ozone. L'enceinte dans sa totalité forme donc la zone de traitement à l'ozone.

Le produit se retrouve donc convoyé en même temps qu'il est soumis à l'action de l'ozone de telle manière qu'un seul passage du produit dans l'enceinte suffise pour que le produit se retrouve correctement traité.

Ceci permet de mieux contrôler le traitement du produit et notamment de mieux gérer l'action de l'ozone sur le produit. On évite ainsi que l'ozone ne vienne oxyder le produit ce qui en détériorerait les aspects visuels, textuels, olfactifs et gustatifs.

De façon avantageuse, le procédé selon l'invention permet de limiter le nombre de paramètres devant être réglés pour à la fois assurer le traitement du produit et éviter son oxydation par l'ozone ce qui rend ledit procédé plus simple à mettre en œuvre.

Par ailleurs, le procédé selon l'invention permet de réduire les temps de séjour du produit dans l'enceinte. Les inventeurs ont ainsi pu constater que le procédé selon l'invention permettait d'avoir un temps de séjour du produit dans l'enceinte compris entre une dizaine de minutes et une heure avec en moyenne un temps de séjour du produit dans l'enceinte d'environ vingt minutes.

De façon particulière, on injecte de l'ozone sur une portion seulement de la longueur de l'enceinte.

De façon particulière, on injecte de l'ozone dans l'enceinte légèrement en aval de l'entrée de l'enceinte.

De façon particulière, on extrait l'ozone résiduel en excès présent dans l'enceinte par une canalisation de sortie légèrement en amont de la sortie de l'enceinte.

De façon particulière, on injecte de l'eau dans l'enceinte sensiblement au niveau de l'entrée de l'enceinte.

L'invention concerne également un dispositif de traitement en continu d'un produit sous forme de solides divisés comportant :
- une enceinte fermée, l'enceinte étant conformée en un serpentin s'enroulant autour d'un arbre central,
- des moyens d'injection d'ozone dans l'enceinte et un circuit de purge de l'ozone en excès dans l'enceinte, les moyens d'injection et le circuit de purge étant conformés pour générer une atmosphère ozonisée sous pression,
- des moyens de convoyage du produit selon un mouvement continu entre une entrée de l'enceinte et une sortie de l'enceinte de sorte que le produit se trouve continuellement dans l'atmosphère ozonisée lors de son convoyage dans l'enceinte et se trouve expulsé hors de l'enceinte par la sortie de l'enceinte après un unique passage du produit à travers l'enceinte, lesdits moyens comprenant des moyens de vibration faisant vibrer l'enceinte.

D'autres caractéristiques et avantages de l'invention apparaîtront plus clairement à la lumière de la description qui va suivre et des dessins annexés, concernant un mode de réalisation particulier de l'invention.

### BREVE DESCRIPTION DES DESSINS

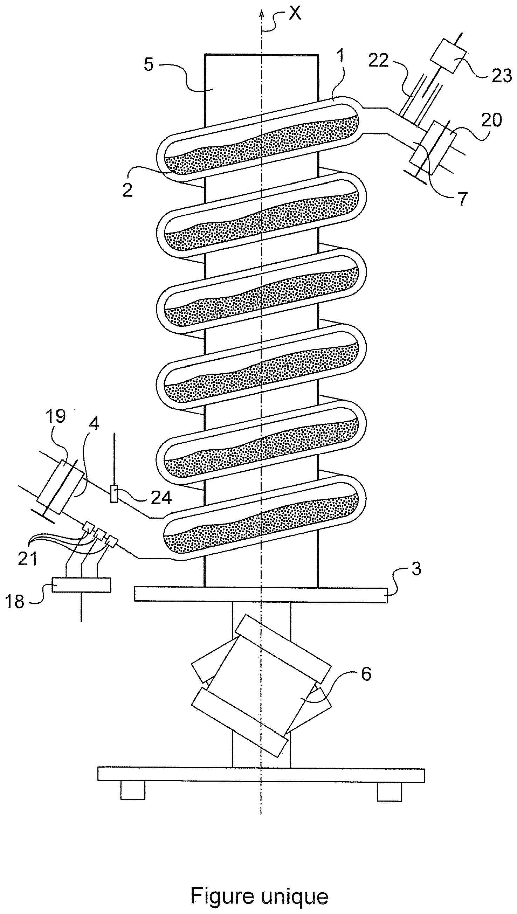

Il sera fait référence à la figure unique annexée qui est une vue en coupe longitudinal schématique d'un dispositif de traitement selon un mode de réalisation particulier de l'invention.

### DESCRIPTION DETAILLEE D'UNE MISE EN ŒUVRE PARTICULIERE DE L'INVENTION

En référence à l'unique figure, le dispositif de traitement selon le mode de réalisation particulier de l'invention, comporte une enceinte 1 délimitant un espace intérieur formant une zone de traitement 2 d'un produit sous forme de solides divisés. Le dispositif de traitement permet ici d'assurer une débactérisation du produit. Cette application n'est bien entendu pas limitative et le dispositif de traitement pourra être mis en œuvre pour assurer une décontamination du produit notamment l'élimination des résidus de pesticides et des mycotoxines.

L'enceinte 1 s'étend longitudinalement selon un axe X. L'enceinte 1 comporte à une première extrémité une entrée 4 et à une deuxième extrémité une sortie 7. L'enceinte 1 est par exemple en acier inoxydable. L'enceinte 1 est ici conformé en un serpentin s'enroulant autour d'un arbre central 5 du dispositif tout en étant fixé audit arbre central 5, l'arbre central 5 s'étendant également selon l'axe X.

Le dispositif comporte en outre des moyens de vibration de l'enceinte 1 afin de provoquer le déplacement du produit de l'entrée 4 de l'enceinte 1 à la sortie 7 de l'enceinte 1.

De façon connue en soi, les moyens de vibration comportent un plateau 3 sur lequel est fixé l'arbre central 5 et des moteurs à balourds 6 agencés pour faire vibrer ledit plateau 3 et donc par-là l'arbre central 5 ensemble l'enceinte 1.

Les moyens de vibration sont par exemple agencés de sorte que le produit demeure dans l'enceinte 1 entre cinq minutes et une heure et de préférence entre cinq minutes et quarante minutes. Alternativement les moyens de vibration sont par exemple agencés de sorte que le produit demeure dans l'enceinte 1 entre une dizaine de minutes et une heure ou de préférence entre une dizaine de minutes et quarante minutes.

Le dispositif comporte des moyens d'injection d'ozone dans l'enceinte 1 qui sont ici raccordés à une source d'alimentation 18 en ozone sous pression. Par « ozone » on entend ici tout aussi bien de l'ozone généré à partir d'oxygène pur que de l'ozone généré à partir d'air atmosphérique séché. Les moyens d'injection d'ozone sont par exemple agencés de sorte à injecter de l'ozone dans l'enceinte 1 à une concentration comprise entre 30 et 90 grammes par mètre cube et de préférence à 60 grammes par mètre cube.

Du fait que le traitement dans l'enceinte 1 s'effectue sous pression et non à la pression atmosphérique, le dispositif et en particulier l'enceinte 1 sont bien entendu conformés pour supporter la pression régnant à l'intérieur de l'enceinte 1. En particulier, le dispositif comporte un sas hermétique de compression 19 agencé en amont de l'entrée 4 de l'enceinte 1 et commandé par des vannes. De même, le dispositif comporte un sas hermétique de décompression 20 agencé en aval de la sortie 7 et commandé par des vannes.

Les moyens d'injection comportent en l'espèce des buses 21 (dont une partie seulement est visible ici) à qui sont agencées en série. De préférence, les premières buses 21 sont agencées au niveau de la première extrémité de l'enceinte 1 légèrement en aval de l'entrée 4 et les dernières buses 21 sont agencées sensiblement au niveau de la première spire de l'enceinte. De la sorte, il s'avère plus aisé de contrôler l'action de l'ozone sur le produit.

Selon une mise en œuvre particulière de l'invention, le dispositif comporte des moyens de pilotage (non représentés ici) des buses 21 qui sont agencés de sorte que chaque buse 21 soit pilotable de manière individuelle. Ceci permettra encore davantage de mieux contrôler l'action de l'ozone sur le produit.

Le dispositif comporte par ailleurs un circuit de purge pour l'extraction de l'ozone en excès dans l'enceinte 1. Le circuit de purge comporte ici une canalisation de sortie 22 agencée au niveau de la deuxième extrémité de l'enceinte 1 et légèrement en amont de la sortie 7 de l'enceinte 1. Le circuit de purge comporte en outre un destructeur d'ozone résiduel 23 (tel qu'un brûleur ou un destructeur catalytique) raccordé à la canalisation de sortie 22 pour détruire l'ozone résiduel en excès extrait de l'enceinte 1.

Ainsi décrit, l'agencement des buses 21 et de la canalisation de sortie 22 permet une circulation de l'ozone dans l'enceinte 1 de même sens que la circulation du produit dans l'enceinte 1. De la sorte, il s'avère plus aisé de contrôler l'action de l'ozone sur le produit.

Les moyens d'injection et le circuit de purge sont ici agencés pour générer une atmosphère ozonisée dans l'enceinte 1 telle que la masse en ozone présente dans l'enceinte 1 soit comprise entre 2 et 18 grammes par kilogramme de produit présent dans l'enceinte 1. De préférence, les moyens d'injection et le circuit de purge sont agencés pour générer une atmosphère ozonisée dans l'enceinte 1 telle que la masse en ozone présente dans l'enceinte 1 soit comprise entre 2 et 10 grammes par kilogramme de produit présent dans l'enceinte 1 et plus préférentiellement entre 3 et 5 grammes par kilogramme de produit présent dans l'enceinte 1. Alternativement, les moyens d'injection et le circuit de purge sont agencés pour générer une atmosphère ozonisée dans l'enceinte 1 telle que la masse en ozone présente dans l'enceinte 1 soit sensiblement de 10 grammes par kilogramme de produit présent dans l'enceinte 1.

En outre, les moyens d'injection et le circuit de purge sont ici conformés pour générer une atmosphère ozonisée dans l'enceinte 1 présentant une pression comprise entre 0.1 et 1 bar. De préférence, les moyens d'injection et le circuit de purge sont agencés pour générer une atmosphère ozonisée dans l'enceinte 1 de 0.6 bar.

Par ailleurs, le dispositif comporte des moyens d'humidification de l'atmosphère ozonisée et par là du produit. Lesdits moyens comportent ici un injecteur d'eau 24 qui est agencé au niveau de la première extrémité 3 de l'enceinte 1 légèrement en aval de l'entrée 4 de l'enceinte 1. L'injecteur d'eau 24 est ici agencé pour se trouver à l'aplomb de certaines des buses 21 d'injection d'ozone. Ceci permet de relativement bien humidifier le produit au moment de son entrée dans l'atmosphère ozonisée.

En complément ou en remplacement, selon le caractère hygroscopique du produit, le dispositif peut comprendre des moyens de pré-humidification de manière homogène du produit qui sont agencés en amont de l'entrée 4 du dispositif.

De la sorte, pour traiter le produit, on introduit le produit dans l'enceinte 1 par l'entrée 4. Le produit se trouve alors humidifié par l'injecteur d'eau 24 et en contact avec l'ozone présent à l'intérieur de l'enceinte 1. Le produit est ainsi convoyé dans l'enceinte 1 à l'aide des moyens de vibration de l'enceinte 1 avant d'être expulsé par la sortie 7.

On constate que tout au long de son unique passage dans l'enceinte 1, le produit convoyé est continuellement en mouvement et soumis à l'atmosphère ozonisée ce qui en favorise le traitement.

On est ainsi parvenu à réaliser un procédé de débactérisation d'un produit par ozone.

L'invention permet par ailleurs avantageusement de restreindre le nombre de paramètres permettant de gérer le traitement du produit. Les paramètres sur lesquels il est possible de jouer pour optimiser le traitement sont ainsi :
- le temps de séjour du produit dans l'enceinte 1 (en agissant sur l'intensité des vibrations émises par les moyens de vibration de l'enceinte 1),
- le taux d'humidité (en agissant sur les moyens d'humidification), et
- le pourcentage en masse d'ozone par masse de produit (en agissant sur les moyens d'injection de l'ozone et le circuit de purge).

L'association pour la première fois du principe de convoyage continu du produit à une exposition simultanée continue à l'ozone procure de multiples avantages et notamment un meilleur contrôle du traitement du produit ainsi qu'une plus grande rapidité de traitement.

L'invention ouvre donc la voie au traitement de produits particulièrement délicats, tels que les épices (poivre, curry, curcuma, etc...), herbes, aromates, les légumes déshydratés, les plantes aromatiques ou médicinales, les fruits secs, les produits laitiers en poudre...

L'invention n'est pas limitée à la mise en œuvre décrite mais englobe au contraire toute variante entrant dans le cadre de l'invention.

Les buses pourront être agencées différemment de ce qui a été décrit. Par exemple, les buses pourront être agencées de sorte à être présentes sur toute la longueur de l'enceinte. Les buses pourront également être présentes uniquement au niveau de la sortie de l'enceinte et le circuit de purge agencé au niveau de l'entrée de l'enceinte de sorte que l'ozone circulera à contre-courant du produit dans l'enceinte.

De même, les moyens d'humidification du produit pourront être différents de ce qui a été décrit. Par exemple, lesdits moyens pourront être agencés en amont de l'entrée de l'enceinte par exemple avant le sas de compression. En complément ou en remplacement, les moyens d'humidification pourront comporter un circuit de brumisation agencé sur toute ou partie de la longueur de l'enceinte pour mieux contrôler le taux d'humidité de l'atmosphère ozonisée régnant dans l'enceinte et donc le taux d'humidité du produit.

Les moyens d'humidification du produit pourront projeter de l'eau ozonisée en place de l'eau décrite pour participer, en coopération avec les moyens d'injection d'ozone, à la création d'une atmosphère ozonisée dans l'enceinte. En outre, bien qu'ici les moyens d'humidification du produit soient distincts des moyens d'injection de l'ozone, ils pourront être confondus, les moyens d'injection projetant alors directement de l'eau ozonisée dans l'enceinte pour y créer une atmosphère ozonisée.

Le dispositif pourra également comprendre des moyens de séchage agencés par exemple au niveau de la sortie de l'enceinte pour mieux contrôler le taux d'humidité de l'atmosphère ozonisée régnant dans l'enceinte et donc le taux d'humidité du produit.

## Revendications

1. Procédé de traitement en continu d'un produit sous forme de solides divisés comportant les étapes de :
- introduire le produit dans une enceinte (1) fermée dans laquelle règne une atmosphère ozonisée sous pression, l'enceinte étant conformée en un serpentin s'enroulant autour d'un arbre central,
- convoyer le produit dans l'enceinte selon un mouvement continu entre une entrée (4) de l'enceinte et une sortie (7) de l'enceinte de sorte que le produit se trouve continuellement dans l'atmosphère ozonisée lors de son convoyage dans l'enceinte, le produit étant convoyé dans l'enceinte par des moyens de vibration (3, 6) faisant vibrer l'enceinte,
- expulser le produit hors de l'enceinte par la sortie de l'enceinte après un unique passage du produit à travers l'enceinte.

2. Procédé selon la revendication 1, dans lequel on injecte de l'ozone sur une portion seulement de la longueur de l'enceinte (1).

3. Procédé selon l'une des revendications précédentes, dans lequel on injecte de l'ozone dans l'enceinte (1) légèrement en aval de l'entrée de l'enceinte.

4. Procédé selon l'une des revendications précédentes, dans lequel on extrait l'ozone résiduel en excès présent dans l'enceinte (1) par une canalisation de sortie (22) légèrement en amont de la sortie (7) de l'enceinte.

5. Procédé selon l'une des revendications précédentes, dans lequel on injecte de l'eau dans l'enceinte (1) sensiblement au niveau de l'entrée (4) de l'enceinte.

6. Dispositif de traitement en continu d'un produit sous forme de solides divisés comportant :
- une enceinte (1) fermée, l'enceinte étant conformée en un serpentin s'enroulant autour d'un arbre central,
- des moyens d'injection d'ozone dans l'enceinte et un circuit de purge de l'ozone en excès dans l'enceinte, les moyens d'injection et le circuit de purge étant conformés pour générer une atmosphère ozonisée sous pression,
- des moyens de convoyage du produit selon un mouvement continu entre une entrée (4) de l'enceinte et une sortie (7) de l'enceinte de sorte que le produit se trouve continuellement dans l'atmosphère ozonisée lors de son convoyage dans l'enceinte et se trouve expulsé hors de l'enceinte par la sortie de l'enceinte après un unique passage du produit à travers l'enceinte, les moyens de convoyage comprenant des moyens de vibration faisant vibrer l'enceinte.

## Patentansprüche

1. Verfahren zur kontinuierlichen Behandlung eines Produktes in Form von geteilten Feststoffen, umfassend die Schritte:
- Einführen des Produktes in eine geschlossene Einfassung (1), in der eine unter Druck stehende ozonhaltige Atmosphäre herrscht, wobei die Einfassung zu einer Schlange geformt ist, die sich um eine zentrale Welle wickelt,
- Fördern des Produktes in der Einfassung mit einer kontinuierlichen Bewegung zwischen einem Einlass (4) der Einfassung und einem Auslass (7) der Einfassung, derart, dass sich das Produkt während seiner Förderung in der Einfassung ständig in der ozonhaltigen Atmosphäre befindet, wobei das Produkt in der Einfassung mittels Vibrationsmitteln (3, 6) gefördert wird, die die Einfassung zum Vibrieren bringen,
- Ausstoßen des Produktes aus der Einfassung durch den Auslass der Einfassung nach einem einzigen Durchlauf des Produktes durch die Einfassung.

2. Verfahren nach Anspruch 1, bei dem man das Ozon nur auf einem Teil der Länge der Einfassung (1) einspritzt.

3. Verfahren nach einem der vorhergehenden Ansprüche, bei dem man das Ozon in die Einfassung (1) geringfügig stromabwärts des Einlasses der Einfassung einspritzt.

4. Verfahren nach einem der vorhergehenden Ansprüche, bei dem man das überschüssige restliche Ozon, das in der Einfassung (1) vorhanden ist, durch eine Auslassleitung (22) geringfügig stromaufwärts des Auslasses (7) der Einfassung entfernt.

5. Verfahren nach einem der vorhergehenden Ansprüche, bei dem man das Wasser im Wesentlichen im Bereich des Einlasses (4) der Einfassung in die Einfassung (1) einspritzt.

6. Vorrichtung zur kontinuierlichen Behandlung eines Produktes in Form von geteilten Feststoffen, umfassend:
- eine geschlossene Einfassung (1), wobei die Einfassung zu einer Schlange geformt ist, die sich um eine zentrale Welle wickelt,
- Ozoneinspritzmittel zum Einspritzen von Ozon in die Einfassung und einen Ableitungskreis für das überschüssige Ozon in der Einfassung, wobei die Einspritzmittel und der Ableitungskreis so geformt sind, dass sie eine unter Druck stehende ozonhaltige Atmosphäre erzeugen,
- Fördermittel zum Fördern des Produktes mit einer kontinuierlichen Bewegung zwischen einem Einlass (4) der Einfassung und einem Auslass (7) der Einfassung, derart, dass sich das Produkt während seiner Förderung in der Einfassung ständig in der ozonhaltigen Atmosphäre befindet und nach einem einzigen Durchlauf des Produktes durch die Einfassung aus der Einfassung ausgestoßen wird, wobei die Fördermittel Vibrationsmittel umfassen, die die Einfassung zum Vibrieren bringen.

## Claims

1. A method of continuously treating a substance in the form of divided solids, the method comprising the steps of:
. introducing the substance into a closed enclosure (1) in which there exists an ozonated atmosphere under pressure, the enclosure being shaped into a coil wound around a central shaft;
. conveying the substance in the enclosure with continuous movement between an inlet (4) of the enclosure and an outlet (7) of the enclosure in such a manner that the substance is located continuously in the ozonated atmosphere while it is being conveyed in the enclosure, the substance being conveyed by vibration means (3, 6) causing the enclosure to vibrate; and
. unloading the substance from the enclosure via the outlet after a single passage of the substance through the enclosure.

2. A method according to claim 1, wherein ozone is injected over a fraction only of the length of the enclosure (1).

3. A method according to any preceding claim, wherein ozone is injected into the enclosure (1) a little downstream from the inlet of the enclosure.

4. A method according to any preceding claim, wherein the excess residual ozone present inside the enclosure (1) is extracted via an outlet duct (22) a little upstream from the outlet (7) of the enclosure.

5. A method according to any preceding claim, wherein water is injected into the enclosure (1) substantially at the inlet (4) of the enclosure.

6. A device for continuously treating a substance in the form of divided solids, the device comprising:
. a closed enclosure (1), the enclosure being shaped into a coil wound around a central shaft;
. injection means for injecting ozone into the enclosure and a purge circuit for purging excess ozone in the enclosure, the injection means and the purge circuit being shaped to generate an ozonated atmosphere under pressure; and
. conveyor means for conveying the substance with continuous movement between an inlet (4) of the enclosure and an outlet (7) of the enclosure in such a manner that the substance is located continuously in the ozonated atmosphere while it is being conveyed in the enclosure and is unloaded from the enclosure via the outlet after a single passage of the substance through the enclosure, said means comprising vibration means causing the enclosure to vibrate.
